# EUROPÄISCHE PATENTANMELDUNG

(11) **EP 1 847 248 A1**
(43) Veröffentlichungstag der Anmeldung: **24.10.2007**
(21) Anmeldenummer: 06008335.9
(22) Anmeldetag: 21.04.2006
(51) Int. Cl.: A61K 8/46, A61Q 5/00, A61Q 5/12, A61Q 19/00

(54) **Kosmetische Mittel zum Schutz oder zur Regenerierung des Haares oder der Haut**

(71) Anmelder: Schrader, Andreas, Dr., 37603 Holzminden (DE)
(72) Erfinder: Schrader, Karlheinz, Dr., 37639 Bevern (DE)
(74) Vertreter: Reitstötter - Kinzebach

(57) **Zusammenfassung**

Die vorliegende Erfindung betrifft ein kosmetisches Mittel, das eine Verbindung der Formel R²R³N-(CH₂)ₙ-SO₂R¹ umfasst, worin R¹ für OR⁴ oder NR⁵R⁶ steht; R² und R³ unabhängig voneinander für H, C₁-C₆-Alkyl, Phenyl-C₁-C₄-alkyl, HCO oder C₁-C₄-Alkyl-CO stehen; R⁴ für C₁-C₆-Alkyl steht; R⁵ und R⁶ unabhängig voneinander für H oder C₁-C₆-Alkyl stehen; und n für 2, 3 oder 4 steht.

Die kosmetischen Mittel sind zum Schutz der Haare und der Haut vor oxidativer Schädigung und zur Regenerierung von oxidativ geschädigten Haaren oder der oxidativ geschädigten Haut brauchbar.

## Beschreibung

Die vorliegende Erfindung betrifft kosmetische Mittel zum Schutz des Haares oder der Haut vor oxidativer Schädigung und zur Regenerierung von oxidativ geschädigtem Haar oder der oxidativ geschädigten Haut.

Wesentlicher Bestandteil des Haares und der Haut ist das Keratin, das maßgeblich die Eigenschaften des Haares bestimmt. Häufig wird das Keratin durch äußere Einflüsse geschädigt. So kommt es durch Sonnenbestrahlung (Bestrahlung mit UV-Licht) zu einer Verringerung der mechanischen Festigkeit des Haares und zu einem Ausbleichen der natürlichen und künstlichen Haarfarbe. Chloriertes Wasser in Schwimmbädern, Pools etc. verursacht Oxidation des Keratins, was zu einem Proteinverlust führen kann. Darüber hinaus wird das Haar häufig chemischen Haarbehandlungen ausgesetzt, wie Behandlungen mit Dauerwell- oder Haarglättungsmitteln, Haarfärbemitteln, Bleichmitteln, Shampoos, etc. Durch diese Behandlungen kann ebenso wie durch UVA-Bestrahlung Wasserstoffperoxid gebildet werden, das zu Interaktionen bzw. farbverändernden Effekten am Haar führen kann. Bleichmittelbelastungen führen in der Regel zu Haarschädigungen, wobei die Melanin-Pigmente in den Cuticula- und den Cortexzellen der Haare durch komplexe chemische Reaktionen abgebaut werden. Dadurch kommt es zu Veränderungen der ursprünglichen Eigenschaften des Haares. Geschädigtes Haar ist rauer, spröder und schwieriger auskämmbar. Es ist poröser und deshalb empfindlicher gegen den Einfluss von Feuchtigkeit und benötigt daher längere Trocknungszeiten. Seine Widerstandsfähigkeit und Reißfestigkeit sind vermindert und seine Dehnungsfähigkeit verändert. Da die bei der Haarbehandlung beteiligten Noxen durch Spülen nicht restlos beseitigt werden können, kommt es letztlich zu schleichender Oxidation und damit zu den genannten mit Farbveränderungen einhergehenden Haarschädigungen.

Es gibt zahlreiche Ansätze, derartige Schädigungen zu begrenzen. Häufig werden hierfür antioxidative Mittel eingesetzt, insbesondere solche, die in der Lage sind, Wasserstoffperoxid abzubauen und aktiven Sauerstoff und freie Radikale abzufangen. Ein derartiges Mittel ist in der US 5,601,806 beschrieben, bei dem eine Aminothiosulfonsäure als Antioxidans verwendet wird.

Dem Stand der Technik ist gemeinsam, dass die Wirkung der bisher bekannten Mittel nicht zufriedenstellend ist oder die Mittel nicht mit kosmetischen Mitteln verträglich sind.

Der vorliegenden Erfindung liegt daher die Aufgabe zugrunde, ein kosmetisches Mittel zur Verfügung zu stellen, das in der Lage ist, das Haar und die Haut vor oxidativer Schädigung wirksam zu schützen und/oder die Schädigung der Haut zu verringern.

Überraschenderweise wurde nun gefunden, dass diese Aufgabe durch ein kosmetisches Mittel gelöst wird, das ein Taurinderivat enthält.

Gegenstand der vorliegenden Erfindung ist daher ein kosmetisches Mittel, umfassend eine Verbindung der Formel I

R²R³N-(CH₂)ₙ-SO₂R¹ (I)

- worin R¹: für OR⁴ oder NR⁵R⁶ steht;
- R² und R³: unabhängig voneinander für H, C₁-C₆-Alkyl, Phenyl-C₁-C₄-alkyl, HCO oder C₁-C₄-Alkyl-CO stehen;
- R⁴: für C₁-C₆-Alkyl steht;
- R⁵ und R⁶: unabhängig voneinander für H oder C₁-C₆-Alkyl stehen; und
- n: für 2, 3 oder 4 steht,
oder ein Salz davon.

Vorzugsweise steht R¹ für NR⁵R⁶, wobei R⁵ und R⁶ die oben genannten Bedeutungen besitzen und insbesondere für H stehen.

R² und R³ stehen vorzugsweise für H oder C₁-C₆-Alkyl und insbesondere für H.

n steht insbesondere für 2.

Eine besonders bevorzugte Ausführungsform ist ein kosmetisches Mittel, das Taurinamid der Formel

H₂N-(CH₂)₂-SO₂NH₂

oder ein Salz davon enthält.

Als Salze der Verbindungen der Formel I kommen Salze mit anorganischen oder organischen Säuren in Betracht. Brauchbare anorganische Säuren sind beispielsweise Salzsäure, Bromwasserstoffsäure, Schwefelsäure oder Phosphorsäure. Brauchbare organische Säuren sind beispielsweise Essigsäure, Propionsäure, Milchsäure, Äpfelsäure, Adipinsäure, Asparaginsäure, Citronensäure, Mandelsäure, Fumarsäure, Maleinsäure, Bernsteinsäure, Weinsäure, Methansulfonsäure oder Toluolsulfonsäure.

Die Herstellung der Verbindungen der Formel I, worin R¹ für NR⁵R⁶ steht, kann nach den in der WO 96/34854 und EP 863 133 beschriebenen Verfahren erfolgen. Man geht aus von einer Verbindung der Formel

R^{a}CH₂CH₂SO₂R^{b}

worin R^{a} und R^{b} für ein Halogenatom stehen, R^{b} auch für einen Azidrest stehen kann oder R^{a} und R^{b} zusammen für -O-SO₂-O- stehen. Diese Verbindungen werden mit einer Azidverbindung, beispielsweise ein Alkalimetallazid, umgesetzt, wobei β-Azidoethansulfonylazid erhalten wird, das beispielsweise durch katalytische Hydrierung zu Taurinamid reduziert werden kann. Die Einführung der an die Stickstoffatome gebundenen Alkyl- oder Acylgruppen erfolgt in üblicher Weise durch Umsetzung mit einem Alkylierungsmittel, wie einem Alkylhalogenid, oder einem Acylierungsmittel, wie einem Carbonsäureanhydrid oder Carbonsäurechlorid.

Die Herstellung der Verbindungen der Formel I, worin R¹ für OR⁴ steht, kann ausgehend von den in der EP 863 133 A2 beschriebenen Verbindungen der Formel

HX·H₂NCH₂CH₂SO₂X'

worin X und X' für Halogen, insbesondere CI oder Br stehen, durch Umsetzung mit dem Alkohol R⁴OH und gegebenenfalls Einführung der Alkyl- oder Acylgruppen am Stickstoffatom (siehe oben) erfolgen.

Die Herstellung derjenigen Verbindungen der Formel I, bei denen n für 3 oder 4 steht, kann in analoger Weise erfolgen.

Die erfindungsgemäßen Mittel können als Hautbehandlungsmittel oder Haarbehandlungsmittel formuliert sei und alle auf diesen Gebieten üblicherweise verwendeten Träger-, Wirk- und Hilfsstoffe enthalten. Die Hautbehandlungsmittel liegen insbesondere als Emulsion, Gel, Lotion, Öl oder Lösung vor. Die Haarbehandlungsmittel können als Shampoo, Spülmittel, Gel, Creme, Lotion, Spray, Tinktur oder Lösung formuliert sein. Die erfindungsgemäßen Mittel weisen vorzugsweise einen pH-Wert im Bereich von 2 bis 10, insbesondere 3,5 bis 10 und besonders bevorzugt 3,5 bis 8 auf.

Geeignete Träger und Hilfsstoffe sind beispielsweise:

Lösungsmittel, wie Wasser, mit Wasser mischbare Alkohole, z.B. Ethanol oder Isopropanol und Gemische davon;

Öle, wie Sojaöl, Erdnussöl, Olivenöl, Sonnenblumenöl, Sesamöl, Avocadoöl, Laurylalkohol, Myristylalkohol, Cetylalkohol, Stearylalkohol, Myristinsäure, Stearinsäure, Palmitinsäure, Ölsäure, etc;

Tenside, wie

anionische Tenside, beispielsweise Alkylsulfate, Alkylpolyglykolethersulfate und Ethercarbonsäuren mit 10 bis 18 Kohlenstoffatomen in der Alkylgruppe und bis zu 12 Glykolethergruppen im Molekül;

nicht-ionische Tenside, wie ethoxylierte Fettalkohole mit 8 bis 30 Kohlenstoffatomen, ethoxylierte Fettsäuren mit 8 bis 30 Kohlenstoffatomen und ethoxylierte Alkylphenole mit 8 bis 15 Kohlenstoffen in der Alkylgruppe;

amphotere Tenside, Alkylamidopropylbetaine (Tego® Betain-Reihe), Aminoxide oder Imidazoliniumbetaine (Miranol®-Reihe).

kationische Tenside, wie quaternäre Ammoniumverbindungen, beispielsweise Alkyltrimethylammoniumchloride, wie Cetyltrimethylammoniumchlorid, Stearyltrimethylammoniumchlorid etc., Stoffe, die mindestens eine Esterfunktion und mindestens eine quaternäre Ammoniumgruppe enthalten, beispielsweise Estersalze von Fettsäuren mit Triethanolamin, Alkylamidoamine etc.

Emulgatoren vom O/W- oder W/O-Typ;

kationische Polymere, wie sie beispielsweise in der WO 02/74265 aufgeführt sind, auf die hier in vollem Umfang Bezug genommen wird. Bevorzugte kationische Polymere sind diejenigen der Polyquaternium-Reihe (siehe Fiedler, Encyclopedia of Excipients, 5th edition, 2002, Seite 1273-1276), quaternisierte Cellulosederivate, wie die Handelsprodukte Celquat® und Polymer JR®, kationische Guarderivate, Polysiloxane mit quaternären Gruppen, polymere Dimethyldiallylammoniumsalze (Merquat®-Reihe, siehe Fiedler, Encyclopedia of Excipients, 2002, Seite 1023 und 1024), Copolymere des Vinylpyrrolidons mit quaternisierten Derivaten des Dialkylaminoalkylacrylats und -methacrylats, die unter der Handelsbezeichnung Gafquat® erhältlich sind etc.;

nicht-ionische Polymere, wie Vinylpyrrolidon/Vinylester-Copolymere, Celluloseether, Polyvinylpyrrolidone, Siloxane etc.;

Proteinhydrolysate;

Vitamine;

Verdickungsmittel, wie Guar-Gum, Alginate, Xanthan-Gum, Gummi arabicum, Cellulosederivate etc.

Bezüglich weiterer Komponenten der erfindungsgemäßen Mittel wird auf die einschlägigen Handbücher, beispielsweise die Monographie von K. H. Schrader, Grundlagen und Rezepturen der Kosmetika, 2. Auflage, Hüthig Buchverlag Heidelberg, 1989, verwiesen.

Die erfindungsgemäßen Haarbehandlungsmittel können auch als Färbemittel, Dauerwellmittel oder Mittel zum Glätten der Haare formuliert sein und die dafür erforderlichen Hilfsstoffe enthalten.

Die Verbindungen der Formel I sind in den erfindungsgemäßen Mitteln im Allgemeinen in einer Menge von 0,1 Gew.-% bis 8 Gew.-%, insbesondere 0,5 Gew.-% bis 5 Gew.-% und besonders bevorzugt 0,5 Gew.-% bis 3 Gew.-%, jeweils bezogen auf das Gesamtgewicht des Mittels, enthalten. Die erfindungsgemäßen Mittel werden in üblicher Weise auf das Haar oder die Haut aufgebracht. Wenn die Mittel in Form eines Shampoos oder Konditionierungsmittels vorliegen, werden sie im Allgemeinen mit Wasser ausgespült. Wenn sie als Konditionierungsmittel, Behandlungscreme, Gel oder Schaum vorliegen, können sie auch auf dem Haar verbleiben ohne ausgespült zu werden. Darüber hinaus können die Mittel in zwei oder mehreren Teilen konfektioniert sein, wie das insbesondere bei Dauerwell- und Färbemitteln häufig der Fall ist.

Die erfindungsgemäßen Mittel sind in der Lage, die Bildung von aktiven Sauerstoffspezies (Superoxide, Wasserstoffperoxid, Hydroxyradikale, Singulettsauerstoff, hyperchlorige Säure) und von freien Radikalen (Lipidperoxidradikale, Lipidalkoxyradikale, Lipidradikale), die vor allem durch die Bestrahlung mit sichtbarem Licht und UV-Licht oder durch Haarbehandlung mit oxidierenden Mitteln entstehen, zu unterdrücken oder zu verringern. Sie sind daher zum Schutz des Haares und der Haut vor oxidativer Schädigung oder zur Regenerierung von oxidativ geschädigtem Haar oder oxidativ geschädigter Haut brauchbar. Mit Hilfe der erfindungsgemäßen Mittel können farbverändernde Schädigungen des Haares und der Haut vermieden oder verringert werden. Außerdem wird die Schädigung der Struktur der Haare und der Haut vermieden oder verringert, so dass die natürlichen Eigenschaften von Haar und Haut erhalten bleiben oder zumindest teilweise wieder hergestellt werden. Insbesondere die Widerstandsfähigkeit, Reißfestigkeit, Dehnungsfähigkeit und Kämmbarkeit bleiben erhalten oder werden bei bereits geschädigtem Haar signifikant verbessert.

Gegenstand der Erfindung ist daher auch die Verwendung einer Verbindung der Formel I

R²R³N-(CH₂)ₙ-SO₂R¹ (I)

worin R¹ für OR⁴ oder NR⁵R⁶ steht;
R² und R³ unabhängig voneinander für H, C₁-C₆-Alkyl, Phenyl-C₁-C₄-alkyl, HCO oder C₁-C₄-Alkyl-CO stehen;
R⁴ für H oder C₁-C₆-Alkyl steht;
R⁵ und R⁶ unabhängig voneinander für H oder C₁-C₆-Alkyl stehen; und
n für 2, 3 oder 4 steht,
oder eines Salzes davon zum Schutz der Haare und der Haut vor oxidativer Schädigung oder zur Regenerierung von oxidativ geschädigten Haaren oder der oxidativ geschädigten Haut oder als Konditionierungsmittel für Haare und Haut.

Zur erfindungsgemäßen Verwendung werden die Verbindungen als kosmetische Mittel wie oben beschrieben eingesetzt.

Die nachfolgenden Beispiele erläutern die Erfindung ohne sie zu begrenzen.

### BEISPIEL 1

Der Effekt von Taurinamidhydrochlorid auf die Regeneration von dauergewelltem Haar wurde untersucht. Dabei wurden drei Strähnen Euro-Haar (naturbelassen, dicht, Farbe 7,0; Firma Kerling) einer Dauerwellbehandlung unterzogen. Die Dauerwelllösung hatte folgende Zusammensetzung:

| | |
|---|---|
| Demineralisiertes Wasser | 74,79 Gew.-% |
| Natriumcarbonat | 7,14 Gew.-% |
| Thioglykolsäure | 9 Gew.-% |
| Ammoniaklösung, 25 %ig | 9 Gew.-% |
| Texapon K12P (Natriumlaurylsulfat) | 0,07 Gew.-% |
| pH | 9,6 |

Die Fixierlösung hatte folgende Zusammensetzung:

| | |
|---|---|
| Demineralisiertes Wasser | 93,7 Gew.-% |
| Wasserstoffperoxid, 30 %ig | 5,7 Gew.-% |
| Turpinal SL (Etidronsäure) | 0,1 Gew.-% |
| Texapon K12P (Natriumlaurylsulfat) | 0,5 Gew.-% |
| pH | 2,6 |

Die Strähnen wurden 1 Minute in einer Schüttelmaschine gewaschen (13,5 % Texapon K12P in demineralisiertem Wasser) und 2 Minuten mit lauwarmem Wasser ausgewaschen. Die Strähnen wurden tropfnass mit Dauerwelllösung getränkt (10 ml für 3 Strähnen). Nach einer Einwirkzeit von 30 Minuten wurde 3 Minuten mit lauwarmem Wasser ausgewaschen. Die Behandlung mit der Dauerwelllösung wurde dreimal wiederholt. Danach wurden die tropfnassen Strähnen mit Fixierlösung getränkt (10 ml für 3 Strähnen). Nach einer Einwirkzeit von 10 Minuten wurde 3 Minuten mit lauwarmem Wasser ausgewaschen.

Zur Bestimmung der Wirkung von Taurinamid wurden folgende Rinse-off-Formulierungen, die unterschiedliche Mengen an Taurinamidhydrochlorid enthielten, eingesetzt:

| Komponenten (Gew.-%) | Formulierung | | | |
|---|---|---|---|---|
| | 1.1 | 1.2 | 1.3 | 1.4 |
| Demineralisiertes Wasser | 91,4 | 91 | 90,5 | 86,5 |
| Lanette 16 (Cetylalkohol) | 8 | 8 | 8 | 8 |
| Texapon K12P (Natriumlaurylsulfat) | 0,5 | 0,5 | 0,5 | 0,5 |
| Taurinamid ·HCl | 0,1 | 0,5 | 1 | 5 |
| NaOH, 10 %ig bis pH | 7,2 | 6,9 | 6,9 | 6,9 |

Die Formulierungen wurden mit einem Pinsel auf einer Glasplatte von beiden Seiten auf die Strähnen aufgetragen (30 g für 3 Strähnen). Nach einer Einwirkzeit von 10 Minuten wurde 3 Minuten mit lauwarmem Wasser ausgewaschen. Danach ließ man die Haare an der Luft trocknen.

Zwei Tage nach der Behandlung wurde die UVA-induzierte Photonenemission (Chemilumineszenz) gemessen. Die Photonenemission ist ein Maß für die Schädigung des Haares. Sie wurde nach der von S. Benard, H. Nerenz, M. Rohr und A. Schrader in IFSCC magazine Vol. 4 (2001), 185-189 beschriebenen Methode gemessen (ICL-H (Induced Chemiluminescence of Human Hair) *A new method for quantitative analysis of hair stress).* Es wurden folgende Ergebnisse erhalten:

| | |
|---|---|
| Schädigung durch Dauerwelle | 555 % |
| 0,1 % Taurinamid | 329 % |
| 0,5 % Taurinamid | 209 % |
| 1 % Taurinamid | 185 % |
| 5 % Taurinamid | 118 % |

Es ist ersichtlich, dass eine Behandlung mit Taurinamid die Schädigung des Haares signifikant verringert.

### BEISPIEL 2

Die in Beispiel 1 beschriebenen Versuche wurden wiederholt, wobei jedoch an Stelle von Taurinamidhydrochlorid 0,1 Gew.-% bzw. 0,5 Gew.-% Taurinhydrochlorid eingesetzt wurden. Die Ergebnisse waren wie folgt:

| | |
|---|---|
| Schädigung durch Dauerwelle nach Behandlung mit | 471 % |
| 0,1 % Taurin | 350 % |
| 0,5 % Taurin | 324 % |

## Patentansprüche

1. Kosmetisches Mittel, umfassend eine Verbindung der Formel I
R²R³N-(CH₂)ₙ-SO₂R¹
worin R¹ für OR⁴ oder NR⁵R⁶ steht;
R² und R³ unabhängig voneinander für H, C₁-C₆-Alkyl, Phenyl-C₁-C₄-alkyl, HCO oder C₁-C₄-Alkyl-CO stehen;
R⁴ für C₁-C₆-Alkyl steht;
R⁵ und R⁶ unabhängig voneinander für H oder C₁-C₆-Alkyl stehen; und
n für 2, 3 oder 4 steht,
oder ein Salz davon.

2. Mittel nach Anspruch 1, wobei R¹ für NR⁵R⁶ steht.

3. Mittel nach Anspruch 2, wobei R⁵ und R⁶ für H stehen.

4. Mittel nach einem der vorhergehenden Ansprüche, wobei R² und R³ für H oder C₁-C₆-Alkyl stehen.

5. Mittel nach einem der vorhergehenden Ansprüche, wobei n für 1 steht.

6. Mittel nach einem der vorhergehenden Ansprüche in Form eines Haarbehandlungsmittels.

7. Verwendung einer Verbindung der Formel I
R²R³N-(CH₂)ₙ-SO₂R¹ (I)
worin R¹ für OR⁴ oder NR⁵R⁶ steht;
R² und R³ unabhängig voneinander für H, C₁-C₆-Alkyl, Phenyl-C₁-C₄-alkyl, HCO oder C₁-C₄-Alkyl-CO stehen;
R⁴ für H oder C₁-C₆-Alkyl steht;
R⁵ und R⁶ unabhängig voneinander für H oder C₁-C₆-Alkyl stehen; und
n für 2, 3 oder 4 steht,
oder eines Salzes davon zum Schutz der Haare und der Haut vor oxidativer Schädigung oder zur Regenerierung von oxidativ geschädigten Haaren oder der oxidativ geschädigten Haut.

8. Verwendung einer Verbindung der Formel I wie in Anspruch 7 definiert als Konditionierungsmittel für Haare und Haut.

9. Verwendung nach Anspruch 7 oder 8, wobei R¹ für OH oder NR⁵R⁶ steht.

10. Verwendung nach Anspruch 9, wobei R¹ für NR⁵R⁶ steht und R⁵ und R⁶ für H stehen.

11. Verwendung nach einem der Ansprüche 7 bis 10, wobei R² und R³ für H oder C₁-C₆-Alkyl stehen.

12. Verwendung nach einem der Ansprüche 7 bis 11, wobei n für 2 steht.
